# EUROPÄISCHE PATENTANMELDUNG

(11) **EP 2 450 703 A1**
(43) Veröffentlichungstag der Anmeldung: **09.05.2012**
(21) Anmeldenummer: 10190396.1
(22) Anmeldetag: 08.11.2010
(51) Int. Cl.: G01N 31/12, G01N 33/18

(54) **Vorrichtung und Verfahren zur gemeinsamen Bestimmung von Ammoniumsticksoff, Gesamtstickstoff und gesamtem organischem Kohlenstoff**

(71) Anmelder: FATAX GmbH, 53332 Bornheim (DE)
(72) Erfinder: Faßbender, Franz Theo, 53332, Bornheim (DE)
(74) Vertreter: von Kreisler Selting Werner

(57) **Zusammenfassung**

Die Erfindung betrifft eine Vorrichtung zur gemeinsamen Bestimmung von Ammoniumsticksoff, Gesamtstickstoff und gesamten organischen Kohlenstoff sowie ein Verfahren zur Bestimmung der genannten Komponenten in einer Probe.

## Beschreibung

Die Erfindung betrifft eine Vorrichtung zur gemeinsamen Bestimmung von Ammoniumsticksoff, Gesamtstickstoff und gesamten organischen Kohlenstoff sowie ein Verfahren zur Bestimmung der genannten Komponenten in einer Probe.

### Hintergrund der Erfindung

Die Bestimmung von Ammoniumstickstoff (NH₄-N), Gesamtstickstoff (Ges.-N) und total organic carben (TOC) bzw. not purgable organic carbon (NPOC) in einer Analyseprobe ist wesentlich für die Steuerung von Kläranlagen und für Betreiber biologischer Kläranlagen von großer Bedeutung. Aus den ermittelten Konzentrationen der genannten Parameter im Einlauf der Kläranlage und der Zulaufmenge in die Abwasserbehandlung können die Zulauffrachten berechnet werden. Ist die aktuelle Leistungsfähigkeit der biologischen Kläranlage in Bezug auf Kohlenstoff- und Stickstoffelimination bekannt, kann somit eine gezielte Zugabe in das System erfolgen. Es kann bei Bedarf auch eine Dosierung von Substrat erfolgen um hohe Eliminationsleistungen aufrecht zu erhalten bzw. Überlastungen zu verhindern. Überlastungen der Anlage führen möglicherweise zu Überschreitungen der Grenzwerte im Ablauf der Abwasserbehandlungsanlage verbunden mit erhöhten Gebühren für die Abwasserabgabe. Hierbei ist besonders die Nitrifikation als wichtiger und empfindlicher Teilprozess zu nennen. Aus den gewonnenen Daten kann auch das für diesen Teilprozess wichtige Kohlenstoff Stickstoffverhältnis (C: N) berechnet werden.

Eine gut funktionierende biologische Kläranlage (hoher Eliminationsgrad für Kohlenstoff- und Stickstoffverbindungen) führt zu Verringerung der eingeleiteten Frachten und damit Verbunden zu einer Verringerung der Eutrophierung des Gewässers. Eine große Bedeutung kommt dem genannten Systems somit im Bereich der Online-Analytik zu.

Zur Bestimmung der genannten Komponenten werden zurzeit Nachweisverfahren eingesetzt, die mindestens zwei getrennte Analysevorrichtungen (Analysatoren) erfordern, die die zur Bestimmung erforderlichen unterschiedlichen Analyseschritte durchführen. Es besteht daher das prinzipielle Bedürfnis für ein vereinfachtes Nachweissystem in dem die erforderlichen Analyseschritte gemeinsam durchgeführt werden können.

### Kurzbeschreibung der Erfindung

Es wurde nunmehr gefunden, dass in einer speziellen Analysevorrichtung mit zwei Reaktoren die Bestimmung von Ammoniumstickstoff, Gesamtstickstoff und gesamten organischen Kohlenstoff (Total Organic Carbon; TOC) innerhalb einer kurzen Analysenzeit durchgeführt werden kann. Die Bestimmung kann sowohl in einem Labor- als auch in einem Online-Analysator erfolgen. Die Erfindung betrifft somit
(1) eine Analysevorrichtung zur Bestimmung von Ammoniumsticksoff, Gesamtstickstoff und gesamten organischen Kohlenstoff in einer Analyseprobe, die eine Ofeneinheit umfassend zwei Öfen, einem ersten Ofen mit einem zur Freisetzung von Ammoniak geeigneten Katalysator gefüllten ersten Reaktor und einem zweiten Ofen mit einem mit einem Oxidationskatalysator gefüllten zweiten Reaktor, Injektionsports, die dem ersten und zweiten Reaktor vorgeschaltet sind und Detektoren für Kohlendioxid und Stickoxide, umfasst, wobei eine Trägergaszuleitung am Kopf des ersten Reaktors vorgesehen ist, eine Trägergasableitung vom Fuß des ersten Reaktors zum Kopf des zweiten Reaktors führt, und die Trägergasableitung vom Fuß des zweiten Reaktors zu den Detektoren für Kohlendioxid und Stickoxide führt;
(2) ein Verfahren zur Bestimmung von Ammoniumsticksoff, Gesamtstickstoff und gesamten organischen Kohlenstoff in einer Probe, vorzugsweise in einer Analysevorrichtung von Aspekt (1), umfassend
   (a) Injektion der zu analysierenden Probe in einen mit zur Freisetzung von Ammoniak geeigneten Katalysator gefüllten ersten Reaktor unter Freisetzen von Ammoniak aus der Probe,
   (b) Weiterleiten des Reaktionsgemisches von (a) in einem inerten Trägergasstrom in einen mit einem Katalysator zur Oxidation von Kohlenstoff- und Stickstoffverbindungen gefüllten zweiten Reaktor und Oxidation des Ammoniaks zu Stickoxiden,
   (c) Weiterleiten des Reaktionsgemisches von (b) in dem inerten Trägergasstrom zu einem Detektor für Stickoxide und Bestimmung dessen Gehalt an Stickoxiden,
   (d) Injektion der gleichen zu analysierenden Probe in den zweiten Reaktor (7) und Oxidation der in der Probe enthaltenen Kohlenstoff- und Stickstoffverbindungen zu Kohlendioxid und Stickoxiden, und
   (e) Weiterleiten des Reaktionsgemisches von (d) in einem inerten Trägergasstrom zu einem Detektor für Stickoxide und einem Detektor für Kohlendioxid und Bestimmung dessen Gehalt an Stickoxiden und an Kohlendioxid; und
(3) die Verwendung einer Analysevorrichtung von Aspekt (1) zur Bestimmung von Ammoniumsticksoff, Gesamtstickstoff und gesamten organischen Kohlenstoff in einer Probe.

### Kurzbeschreibung der Figur

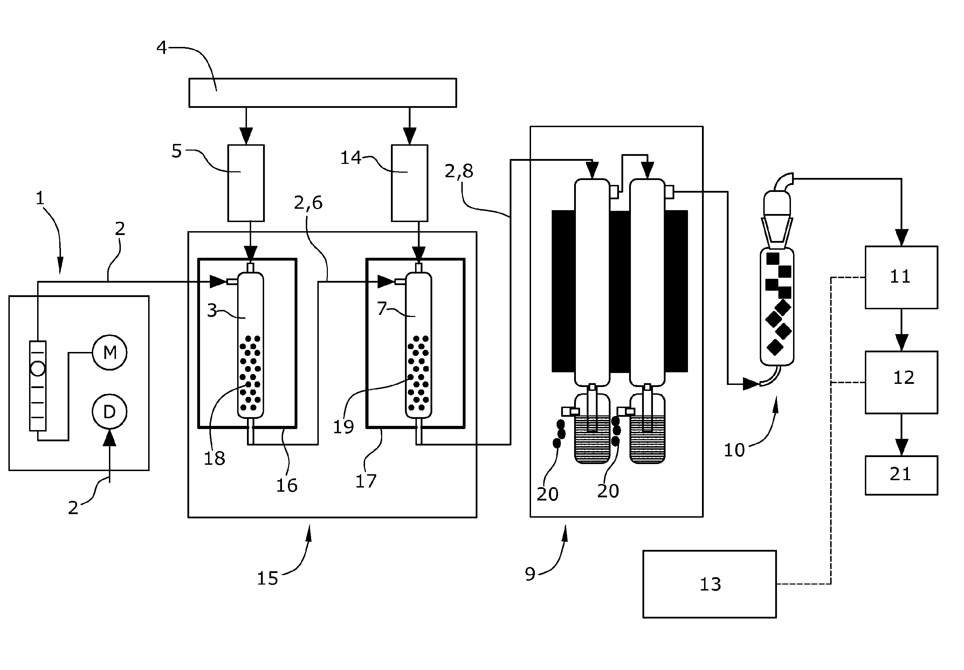

Figur 1 zeigt den schematischen Aufbau der erfindungsgemäßen Analysevorrichtung.

### Detaillierte Beschreibung der Erfindung

Die Analysevorrichtung (Analysator) gemäß Aspekt (1) der Erfindung kann neben den vorstehend genannten Ofeneinheit, Injektionsports und Detektoren noch weitere der folgenden funktionellen Einheiten umfassen: eine Trägergaseinheit, eine Kühlereinheit, eine Absorbereinheit, eine Auswerteeinheit und eine Abgasreinigung.

Die Trägergaseinheit ist dabei der Ofeneinheit vorgeschaltet und versorgt über eine Trägergasleitung die Reaktoren der Ofeneinheit mit Trägergas. Die Kühleinheit und/oder Absorbereinheit sind in der Trägergasleitung vom zweiten Reaktor zu den Detektoren zwischengeschaltet, und die Abgasreinigung ist den Detektoren nachgeschaltet. Bevorzugt dabei ist eine Vorrichtung die alle der genannten funktionellen Einheiten umfasst.

Die Trägergasableitungen, die vom Fuß des ersten Reaktors zum Kopf des zweiten und vom Fuß des zweiten Reaktors zu den Detektoren für Kohlendioxid und Stickoxide führen, ermöglichen fluidische Verbindungen der entsprechenden Einheiten, in denen mittels des Trägergases die flüchtigen Reaktionsprodukte der in den Reaktoren umgesetzten Proben zum nächsten Reaktor bzw. den Detektoren, über gegebenenfalls vorhandene zwischengeschaltete funktionelle Einheiten befördert werden.

Die einzelnen funktionellen Einheiten der erfindungsgemäßen Analysevorrichtung werden nachfolgend näher erläutert.
1. Trägergaseinheit (1): In der Trägergaseinheit (1) wird ein kontinuierlicher Trägergasstrom aus CO₂-freier synthetischer Luft von 8-10 l/h erzeugt. Der Trägergasstrom (2) dient dem Transport des, im ersten Reaktor (3) aus der in diesen über den Injektionsport 1 (5) eingebrachten Abwasserprobe (4), frei gesetzten Ammoniaks (6) hin zum zweiten Reaktor (7). Dem anschließenden Transport der Verbrennungsgase (8) in die Einheiten Kühlung (9), Adsorption (10) und in die Detektoren für Kohlendioxid (11) und Stickoxide (12).
2. Injektionsports (5 und 14): Die Injektionsports 1 (5) und 2 (14) dienen zur Aufgabe der Proben (4) in die Reaktoren (3/7) ohne, das es zu einem Verlust von Trägergas (2) und damit zum Verlust von Reaktionsprodukten (6/8) kommt.
3. Ofeneinheit (15): Die Ofeneinheit (15) besteht aus zwei Öfen (16/17) in denen jeweils ein Quarzglasreaktor (3/7) mit Katalysator (18/19) eingesetzt ist. Die Öfen (16/17) können bis 900°C aufgeheizt werden. In den Reaktoren (3/7) laufen bei Verwendung unterschiedlicher Katalysatoren (18/19) bei unterschiedlichen Temperaturen nach Injektion der Abwasserprobe (4) die Reaktionen ab, bei der es im ersten Reaktor (3) zur Freisetzung vom Ammoniak (6) und im zweiten Reaktor (7) zur thermisch katalytischen Oxidation von Kohlenstoff zu Kohlendioxid, von Stickstoffverbindungen zu Stickoxiden und anderen Reaktionsprodukten (8) kommt.
4. Kühlereinheit (9): In der Kühlereinheit (9) wird der in den Reaktoren entstandene Wasserdampf durch Kühlung zu Wasser (20) kondensiert um eine fehlerhafte Detektion der Komponenten Kohlendioxid und Stickstoffoxid zu verhindern.
5. Absorbereinheit (10): In der Absorbereinheit (10) erfolgt die Absorption der, bei der Verbrennung entstandenen, unerwünschten Nebenprodukte an den eingesetzten Absorbermaterialien Bronze und Zink.
6. Detektor für Kohlendioxid (11): Zur Detektion von Kohlendioxid wird vorzugsweise ein NDIR-Detektor eingesetzt.
7. Detektor für Stickoxide (12): Zur Detektion der Stickoxide kommen Chemolumineszensdetektoren (CLD) bzw. elektrochemische Messzellen zum Einsatz. Die Detektoren können, im Verhältnis zum Trägergasstrom, seriell zueinander geschaltet sein.
8. Auswerteeinheit (13): Die von den Detektoren erzeugten Messsignale werden in einer Auswerteeinheit (13) aufgenommen und mit Messsignalen aus Kalibriervorgängen verglichen und unter Verwendung der Menge an aufgegebener Abwasserprobe die Konzentration der jeweiligen Komponente in mg/l bestimmt.
9. Abgasreinigung (21): Die Abgasreinigung wird nur bei der Verwendung von Ozon in den CLD eingesetzt.

Die erfindungsgemäße Analysevorrichtung ist zur Bestimmung von Ammoniumstickstoff, Gesamtstickstoff und TOC/NPOC geeignet und ermöglicht die Bestimmung der genannten Parameter in einem Analysator innerhalb von sehr kurzen Analysenzeiten, somit ist nur ein Analysensystem zu betreuen.

Die Bestimmung von Ammoniumstickstoff, Gesamtstickstoff, TOC/NPOC in dem Verfahren gemäß Aspekt (2) der Erfindung erfolgt in einem Analysator mit 2 Reaktoren wie vorstehend beschrieben innerhalb einer kurzen Analysenzeit. Durch Injektion der angesäuerten und ausgeblasenen Probe in den ersten Reaktor, der mit alkalischem Trägermaterial gefüllt ist, erfolgt die Freisetzung von Ammoniak bei 160-180 °C. Mit Hilfe des Trägergasstromes erfolgt die Weiterleitung in den zweiten Reaktor, der mit einem Katalysator zur Oxidation von Kohlenstoff- und Stickstoffverbindungen gefüllt ist. Hier erfolgt die Oxidation des Ammoniaks bei 700-900 °C zu Stickoxiden. Die erhaltene Konzentration an Stickoxiden wird mit einem CLD bzw. einem elektrochemischen Sensor erfasst. Durch eine zweite Injektion der gleichen Probe direkt in den zweiten Reaktor werden die enthaltenen Kohlenstoff- und Stickstoffverbindungen zu Kohlendioxid und Stickoxiden oxidiert. Die erhaltene Konzentration an Kohlendioxid wird mit Hilfe eines NDIR-Detektors und die Konzentration an Stickoxiden wird mit einem CLD bzw. einem elektrochemischen Sensor erfasst. Mit der Auswerteeinheit werden die Konzentrationen der einzelnen Parameter berechnet, kalibriert und die absolute Konzentration in der Probe bestimmt.

Die Erfindung wird anhand des nachfolgenden Beispiels näher erläutert, dieses soll den Schutzbereich der Erfindung jedoch nicht einschränken.

### Beispiel

Probenvorbereitung: Die zu analysierende Abwasserprobe (4) wird mit verdünnter Salzsäure auf einen pH-Wert < 3 angesäuerte und mit einem CO₂ freien Luftstrom so lange ausgeblasen bis die in der Abwasserprobe enthaltenen anorganischen Kohlenstoffverbindungen (Carbonat und Hydrogencarbonat) vollständig entfernt wurden. Das Ansäuern der Abwasserprobe verhindert zusätzlich das Austreiben von Ammoniak bei alkalischem pH-Wert der Probe, der Ammoniak wird in Ammoniumchlorid überführt.

Analysenschritt 1: In den Reaktor 1 (3) werden je nach zu erwartender Konzentration von 25 bis 250 µl vorzugsweise 100 bis 150 µl der so vorbehandelten Probe injiziert. Der Reaktor ist mit einem mit Natronlauge konditioniertem Trägermaterial (Katalysator 18) z.B. Bimsstein mit einer Körnung von 1 - 3 mm gefüllt. Der Reaktor (3) wird mit Hilfe des Ofens (16) auf 160-180°C geheizt. Das Wasser wird im Reaktor (3) verdampft und durch den alkalischen pH-Wert die Ammoniumverbindungen in freien Ammoniak überführt. Der Wasserdampf (8) und der entstehende Ammoniak(6) werden mit dem Trägergasstrom (2) in den zweiten Reaktor (7) geleitet. Der zweite Reaktor (7) ist mit einem Katalysator (19) z.B. Platin auf Aluminiumoxid zur Oxidation von Kohlenstoff und Stickstoff gefüllt. Der Reaktor (7) wird mit Hilfe des Ofens (17) auf 700 bis 900°C geheizt. In diesem Analysenschritt erfolgt die Verbrennung des Ammoniaks (6) zu Stickoxiden (8). Die gebildeten Stickoxide (8) werden mit dem Trägergasstrom (2) nacheinander dem Kühler (9), Absorber (10) und den Detektoren (11/12) zugeführt. Mit der Auswerteeinheit (13) wird die Konzentration an Ammoniumstickstoff in der eingesetzten Probe berechnet. Die Konditionierung des Trägermaterials im ersten Reaktor muss durch Injektion von verdünnter Natronlauge nach einigen Analysen (je nach Bedarf) wiederholt werden.

Analysenschritt 2: Nun werden je nach zu erwartender Konzentration 25 bis 250 µl, vorzugsweise 100 bis 150 µl der angesäuerten und ausgeblasenen Probe in den Ofen 2 (17) injiziert. Es erfolgt nach Verdampfung des Wassers die thermisch katalytische Oxidation aller in der Abwasserprobe enthaltenen Kohlenstoff und Stickstoffverbindungen zu Kohlendioxid, Stickoxiden und Nebenprodukten (8). Die gebildeten Oxide und gebildeten Nebenprodukte werden mit dem Trägergasstrom (2) nacheinander dem Kühler (9), Absorber (10) und den Detektoren (11/12) zugeführt. Mit der Auswerteeinheit (13) wird die Konzentration an Kohlenstoff und Gesamtstickstoff in der eingesetzten Probe berechnet. Als Analysenergebnis kann die Konzentration an Ammoniumstickstoff (Ergebnis aus Analysenschritt 1), Gesamtstickstoff und organischem Kohlenstoff (aus Analysenschritt 2) angegeben werden. Sind in der Abwasserprobe keine bzw. nur geringe Nitrit- und Nitratstickstoffverbindungen enthalten, so ergibt die Differenz aus Gesamt- und Ammoniumstickstoff den organisch gebundenen Stickstoff.

## Patentansprüche

1. Analysevorrichtung zur Bestimmung von Ammoniumsticksoff, Gesamtstickstoff und gesamten organischen Kohlenstoff in einer Analyseprobe, die eine Ofeneinheit (15) umfassend zwei Öfen (16,17), einem ersten Ofen (16) mit einem mit zur Freisetzung von Ammoniak geeigneten Katalysator (18) gefüllten ersten Reaktor (3) und einem zweiten Ofen (17) mit einem mit einem Oxidationskatalysator (19) gefüllten zweiten Reaktor (7),
Injektionsports (5,14), die dem ersten und zweiten Reaktor (3,7) vorgeschaltet sind und
Detektoren für Kohlendioxid und Stickoxide (11,12)
umfasst, wobei eine Trägergaszuleitung am Kopf des ersten Reaktors vorgesehen ist, eine Trägergasableitung vom Fuß des ersten Reaktors zum Kopf des zweiten Reaktors führt, und die Trägergasableitung des zweiten Reaktors (7) zu den Detektoren für Kohlendioxid und Stickoxide (11,12) führt.

2. Analysevorrichtung nach Anspruch 1, die weiterhin eine oder mehrere der folgenden funktionellen Einheiten umfasst: eine der Ofeneinheit (15) vorgeschaltet Trägergaseinheit (1), die über eine Trägergasleitung die Reaktoren(3,7) der Ofeneinheit (15) mit Trägergas (2) versorgt; Kühlereinheiten (9) und Absorbereinheiten (10), die vorzugsweise in der Trägergasleitung vom zweiten Reaktor (7) zu den Detektoren (11,12) zwischengeschaltet sind; Abgasreinigungseinheiten (21), die den Detektoren (11,12) nachgeschaltet sind; und Auswerteeinheiten (13), wobei eine Vorrichtung, die alle der genannten funktionellen Einheiten umfasst, besonders bevorzugt ist.

3. Analysevorrichtung nach Anspruch 1 oder 2, wobei in der Ofeneinheit (15)
(i) die Ofen (16,17) unabhängig voneinander bis auf 900 °C aufheizbar sind;
(ii) Die Reaktoren (3,7) Quarzglasreaktoren sind;
(iii) der zur Freisetzung von Ammoniak geeigneten Katalysator (18) des ersten Reaktors (3) ausgewählt ist aus alkalischen Trägermaterialien, insbesondere einem mit Natronlauge konditionierten Trägermaterial wie Bimsstein oder Aluminiumoxid; und/oder
(iv) der Oxidationskatalysator (19) des zweiten Reaktors (7) ein Platin auf Aluminiumoxid Katalysator ist.

4. Analysevorrichtung nach einem oder mehreren der Ansprüche 1 bis 3, wobei
(i) die Detektoren für Kohlendioxid und Stickoxide (11,12) parallel oder seriell angeordnet sind;
(ii) der Detektor für Kohlendioxid (11) ein Non-Disperse-Infra-Red (NDIR)-Detektor ist;
(iii) der Detektor für Stickoxide (12) ausgewählt ist aus Chemolumineszensdetektoren (CLD) und elektrochemischen Messzellen, insbesondere ein CLD ist.

5. Analysevorrichtung nach einem oder mehreren der Ansprüche 1 bis 4, wobei die Injektionsports (5,14) die Aufgabe der Proben (4) in die Reaktoren (3,7) ohne Verlust von Trägergas (2) und Verlust von Reaktionsprodukten (6/8) ermöglichen.

6. Analysevorrichtung nach einem oder mehreren der Ansprüche 2 bis 5, wobei
(i) die Trägergaseinheit (1) geeignet ist, einen kontinuierlicher Trägergasstrom aus CO₂-freier synthetischer Luft, vorzugsweise mit einem Fluss von 8-10 l/h zu erzeugen;
(ii) die Kühlereinheit (9) zur Kondensation des in den Reaktoren (3,7) entstandenen Wasserdampfes geeignet ist; und/oder
(iii) die Absorbereinheit (10) zur Absorption der bei der Verbrennung in den Reaktoren (3,7) entstandenen, unerwünschten Nebenprodukte einschließlich Halogenwasserstoffe und Schwefeloxide geeignet ist, wobei das Absorbens vorzugsweise Bronze und Zink Materialien umfasst.

7. Verfahren zur Bestimmung von Ammoniumsticksoff, Gesamtstickstoff und gesamten organischen Kohlenstoff in einer Probe (4), vorzugsweise in einer Analysevorrichtung gemäß einem oder mehreren der Ansprüche 1 bis 6, umfassend
(a) Injektion der zu analysierenden Probe (4) in einen mit zur Freisetzung von Ammoniak geeigneten Katalysator (18) gefüllten ersten Reaktor (3) unter Freisetzen von Ammoniak aus der Probe (4),
(b) Weiterleiten des Reaktionsgemisches (6) von (a) in einem inerten Trägergasstrom (2) in einen mit einem Katalysator (19) zur Oxidation von Kohlenstoff- und Stickstoffverbindungen gefüllten zweiten Reaktor (7) und Oxidation des Ammoniaks zu Stickoxiden,
(c) Weiterleiten des Reaktionsgemisches (8) von (b) in dem inerten Trägergasstrom (2) zu einem Detektor für Stickoxide (12) und Bestimmung dessen Gehalt an Stickoxiden,
(d) Injektion der gleichen zu analysierenden Probe (4), in den zweiten Reaktor (7) und Oxidation der in der Probe (4) enthaltenen Kohlenstoff- und Stickstoffverbindungen zu Kohlendioxid und Stickoxiden, und
(e) Weiterleiten des Reaktionsgemisches (8) von (d) in einem inerten Trägergasstrom (2) zu einem Detektor für Stickoxide (12) und einem Detektor für Kohlendioxid (11) und Bestimmung dessen Gehalt an Stickoxiden und an Kohlendioxid.

8. Verfahren nach Anspruch 7, weiterhin umfassend als Schritt (f) die Bestimmung des absoluten Ammoniumstickstoff-, Gesamtstickstoff- und des gesamten organischen Kohlenstoffgehaltes in der Probe (4) durch Normierung der in Schritten (c) und (e) bestimmten relativen Werte.

9. Verfahren nach Anspruch 7 oder 8, wobei
(i) die Probe (4) vor der Injektion angesäuerten und ausgeblasen wird;
(ii) die Temperatur des ersten Reaktors (3), für die Freisetzung von Ammoniak in Schritt (a) 160 bis 180 °C beträgt;
(iii) die Oxidation des Ammoniaks (6) zu Stickoxiden in Schritt (b) und die Oxidation der Probe (4) zu Stickoxiden und Kohlendioxid in Schritt (d) bei 700 bis 900 °C erfolgt;
(iv) die Bestimmung des Gehalts an Stickoxiden in Schritt (c) und (e) mittels eines Chemolumineszensdetektors oder einer elektrochemischen Messzelle erfolgt; und/oder
(v) die Bestimmung des Gehalts an Kohlendioxid in Schritt (e) mittels eines NDIR-Detektors erfolgt.

10. Verwendung einer Analysevorrichtung gemäß einem oder mehreren der Ansprüche 1 bis 6 zur Bestimmung von Ammoniumsticksoff, Gesamtstickstoff und gesamten organischen Kohlenstoff in einer Probe (4).
